# EUROPÄISCHE PATENTANMELDUNG

(11) **EP 1 052 241 A1**
(43) Veröffentlichungstag der Anmeldung: **15.11.2000**
(21) Anmeldenummer: 00110116.1
(22) Anmeldetag: 10.05.2000
(51) Int. Cl.: C07C 209/18

(54) **Verfahren zur Herstellung von aromatischen Aminen**

(30) Priorität: 11.05.1999 DE 19921782
(71) Anmelder: BASF AKTIENGESELLSCHAFT, 67056 Ludwigshafen (DE)
(72) Erfinder: Breuer, Klaus, Dr., 67122 Altrip (DE); Baier, Michael, Dr., 68161 Mannheim (DE); Müller, Ulrich, Dr., 67435 Neustadt/Weinstrasse (DE); Dernbach, Matthias, Dr., 69221 Dossenheim (DE); Rieber, Norbert, Dr., 68259 Mannheim (DE); Hoffmann, Werner, Dr., 67141 Neuhofen (DE)
(74) Vertreter: Isenbruck, Günter, Dr.

(57) **Zusammenfassung**

Die Herstellung von aromatischen Aminen erfolgt durch
(5) Gasphasen-Hydroxylierung von aromatischen C₆₋₁₄-Kohlenwasserstoffen, die zusätzlich durch 1 bis 3 C₁₋₆-Alkylreste und/oder Halogenatome substituiert sein können, mit N₂O an einem Katalysator, und ohne weitere Reinigung des hydroxylierten Produktes nachfolgende
(6) Gasphasen-Aminierung des hydroxylierten Produktes aus Stufe (1) mit Ammoniak.

## Beschreibung

Die Erfindung betrifft ein Verfahren zur Herstellung von aromatischen Aminen, insbesondere zur Herstellung von Anilin aus Benzol.

Anilin kann großtechnisch durch unterschiedliche Verfahren hergestellt werden. Beispielsweise kann Anilin durch Reduktion von Nitrobenzol, entweder mit Eisenfeilspänen und Salzsäure oder katalytisch erzeugtem Wasserstoff, hergestellt werden.

Anilin kann auch durch Ammonolyse von Phenol erhalten werden. Dabei wird Phenol mit Ammoniak in Gegenwart eines Katalysators umgesetzt. Ein entsprechendes Verfahren, das an silicium- und aluminiumhaltigen Katalysatoren durchgeführt wird, ist in US 3,860,650 beschrieben.

Das benötigte Phenol wird in der Regel durch Spaltung von Cumolhydroperoxid nach Hock erhalten. Es kann weiterhin aus Benzol über Benzolsulfonsäure oder über Chlorbenzol und deren Behandlung mit Alkali, aus Toluol über Benzoesäure und aus Cyclohexan über Cyclohexanon/Cyclohexanol hergestellt werden.

Zudem ist es bekannt, Phenol durch Hydroxylierung von Benzol mit N₂O in Gegenwart eines Katalysators herzustellen. Entsprechende Verfahren sind beispielsweise in EP-A-0 406 050 beschrieben.

Vor einer Aminierung wird Phenol dabei in der Regel aufgereinigt und als Reinsubstanz eingesetzt.

Aufgabe der vorliegenden Erfindung ist die Bereitstellung eines Verfahrens zur Herstellung von aromatischen Aminen aus aromatischen Kohlenwasserstoffen und insbesondere zur Herstellung von Anilin aus Benzol, das wirtschaftlich vorteilhaft und unaufwendig durchzuführen ist.

Die Aufgabe wird erfindungsgemäß gelöst durch ein Verfahren zur Herstellung von aromatischen Aminen durch
(1) Gasphasen-Hydroxylierung von aromatischen C₆₋₁₄-Kohlenwasserstoffen, die zusätzlich durch 1 bis 3 C₁₋₆-Alkylreste und/oder Halogenatome, insbesondere Chlor- oder Fluoratome substituiert sein können, mit N₂O an einem, vorzugsweise zeolithischen, Katalysator, und ohne weitere Reinigung des hydroxylierten Produktes nachfolgende
(2) Gasphasen-Aminierung des hydroxylierten Produktes aus Stufe (1) mit Ammoniak, vorzugsweise an einem zeolithischen Katalysator bzw. silicium- und aluminiumhaltigen Katalysator.

Die beiden Teilreaktionen sind dabei an sich bekannt. Für die Aromatenhydroxylierung mit N₂O kann beispielsweise auf Catalysis Today 41 (1998), 365 ― 385 und Applied Catalysis A: General, 98 (1993), 1 ― 20 verwiesen werden. Es wurde erfindungsgemäß gefünden, daß das aus der Gasphasen-Hydroxylierung von aromatischen Kohlenwasserstoffen erhaltene Produkt ohne weitere Reinigung oder Aufarbeitung in eine Gasphasen-Aminierung eingeführt werden kann. Der Begriff 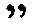 Reinigung" bedeutet die Behandlung des, gegebenenfalls vom Ausgangsstoff befreiten, Rohproduktes der Stufe (1) zur Reindarstellung der zum Beispiel phenolischen Verbindung. Es wird zum Beispiel keine Feindestillation des Phenols vor Stufe (2) durchgeführt. Dabei kann das in Stufe (1) erhaltene Reaktionsprodukt zunächst zwischenkondensiert und gegebenenfalls von überschüssigem Ausgangsstoff befreit und vor der Gasphasen-Aminierung wieder verdampft werden. Vorzugsweise wird das in Stufe (1) erhaltene gasförmige hydroxylierte Produkt jedoch direkt gasförmig in die Stufe (2) geführt, wodurch ein Abkühlen und erneutes Erhitzen vermieden wird.

Als aromatische Kohlenwasserstoffe werden allgemein aromatische C₆₋₁₄-Kohlenwasserstoffe eingesetzt, die zusätzlich durch 1 bis 3 C₁₋₆-Alkylreste oder Halogenatome wie Chlor- oder Fluoratome substituiert sein können. Bei den aromatischen Kohlenwasserstoff-Grundgerüsten handelt es sich dabei um Benzol, Naphthalin, Anthracen oder Phenanthren. Sie sind vorzugsweise durch einen oder zwei C₁₋₆-, vorzugsweise C₁₋₃-Alkylreste oder Halogenatome wie Chlor- oder Fluoratome substituiert. Vorzugsweise liegen keine Substituenten oder ein oder zwei Methyl- oder Ethylreste vor. Besonders bevorzugt wird Benzol eingesetzt, das nach der Umsetzung in den Stufen (1) und (2) Anilin ergibt.

Die Umsetzung mit N₂O erfolgt vorzugsweise in einem Molverhältnis von Kohlenwasserstoff zu N₂O von 1 : 20 bis 50 : 1, besonders bevorzugt 1 : 1 bis 15 : 1 . Die Temperatur bei der Umsetzung beträgt vorzugsweise 250 bis 500°C, besonders bevorzugt 300 bis 450°C. Der Druck bei der Umsetzung beträgt vorzugsweise 0 bis 30 bar, besonders bevorzugt 0 bis 10 bar. Bei der Umsetzung von Benzol wird insbesondere bei einer Temperatur von 330 bis 450°C und einem Druck von 0 bis 10 bar gearbeitet. Neben den Kohlenwasserstoffen und N₂O können noch inerte Trägergase wie Stickstoff in die Umsetzung geführt werden. Dabei kann das Volumenverhältnis von Trägergas zu N₂O 0 bis 50, vorzugsweise 1 bis 20 betragen.

Bei der Gasphasen-Aminierung in Stufe (2) wird vorzugsweise mit einem Molverhältnis von hydroxyliertem Produkt aus Stufe (1) zu Ammoniak von 1 : 2 bis 1 : 100, besonders bevorzugt 1 : 3 bis 1 : 20 gearbeitet. Auch in dieser Stufe kann ein inertes Trägergas wie Stickstoff mitverwendet werden. Das Volumenverhältnis von Trägergas zu Ammoniak beträgt vorzugsweise 1 : 1 bis 50 : 1, besonders bevorzugt 2 : 1 bis 20 : 1.

Sämtliche Reaktionsteilnehmer, das heißt aromatischer Kohlenwasserstoff, N₂O und Ammoniak können bereits zu Beginn in die Stufe (1) geführt werden. Dabei können die Stufen (1) und (2) gleichzeitig durchgeführt werden, was jedoch zu erniedrigten Ausbeuten führen kann. Vorzugsweise wird der aromatische Kohlenwasserstoff erst nach der in Stufe (1) erfolgten Hydroxylierung mit Ammoniak versetzt und in Stufe (2) aminiert. Dazu wird die Umsetzung in der Regel in zwei getrennten Reaktoren durchgeführt, wobei Ammoniak mit einer Zwischeneinspeisung in den Gasstrom von Stufe (1) zu Stufe (2) eingespeist wird.

Die Stufen (1) und (2) können im selben Reaktor durchgeführt werden, wie dies insbesondere bei der gleichzeitigen Umsetzung der Fall ist. Es ist jedoch auch möglich, den Reaktoraustrag aus Stufe (1) zwischenzukondensieren und sodann im gleichen Reaktor unter Zusatz von Ammoniak weiter umzusetzen.

Bevorzugt wird die Hydroxylierung der Aromaten mit N₂O und die Aminierung zu dem aromatischen Amin in separaten Reaktoren durchgeführt, wobei der Reaktoraustrag aus der Hydroxylierung mit Ammoniak versetzt und anschließend in den Aminierungsreaktor überführt wird. Der Reaktionsaustrag kann dabei in einer zwischengeschalteten Destillation von überschüssigem bzw. restlichem Ausgangsstoff, zum Beispiel Benzol, befreit werden. Bei einer Hydroxylierung und Aminierung in einem Reaktor ist das Katalysatorbett sowohl mit dem Hydroxylierungskatalysator als auch mit dem Aminierungskatalysator bestückt. Diese Katalysatoren können auch identisch sein, d. h. im Reaktor befindet sich nur eine Aktivmasse. Das erfindungsgemäße Verfahren wird vorzugsweise kontinuierlich durchgeführt. Dabei wird die Stufe (1) vorzugsweise mit einem Umsatz von 5 bis 50 % bezüglich Ausgangsstoff (Aromat) bzw. 50 bis 100 % bezüglich N₂O, geführt. Aus sicherheitstechnischen Gründen ist in Stufe (1) eine Erzielung eines hohen Umsatzes bezüglich N₂O vorteilhaft, weil in diesem Fall bei Zusatz von Ammoniak vor der Stufe (2) keine prinzipiell explosionsfähigen Mischungen aus N₂O und Ammoniak entstehen können.

Die Umsetzung in den Stufen (1) und (2) kann an unterschiedlichen oder gleichen, vorzugsweise zeolithischen, Katalysatoren durchgeführt werden. Vorzugsweise werden in Stufe (1) und/oder (2) Fe, Ga oder Gemische davon enthaltende bzw. damit dotierte Zeolithe eingesetzt. Die Zeolithe sind dabei vorzugsweise mit Fe, Ga oder Gemischen davon in einer Menge von 0,005 bis 1,5 Gew.-%, insbesondere 0,05 bis 1,0 Gew.-%, bezogen auf den gesamten Katalysator, dotiert bzw. enthalten diese Mengen.

Für die Stufe (2) werden bevorzugt silicium- und aluminiumhaltige Katalysatoren eingesetzt, wie sie z.B. in US 3,860,650 beschrieben sind.

Es können auch beide Stufen mit einem Fe-dotierten hochsilikatischen Zeolith mit dem Strukturtyp MFI, MEL, MOR, BEA, MWW, FER, MTW oder MFI/MEL-Mischstrukturen durchgeführt werden. Besonders bevorzugt wird ein Fe-dotierter Zeolith von Strukturtyp MFI, MEL oder einer MFI/MEL-Mischstruktur eingesetzt.

Derartige Katalysatoren weisen sowohl eine Hydroxylierungsaktivität als auch eine Aminierungsaktivität auf Ein spezieller geeigneter Katalysator ist ein H-(Fe)-MFI. Geeignete Katalysatoren sind in einer Vielzahl von Schriften beschrieben, beispielsweise in EP-A-0 341 165, EP-A-0 406 050, US 5,110,995, WO 95/27560, US 5,672,777, WO 95/27691.

Dabei kann während des regenerativen Abbrennens eines verkokten Hydroxylierungskatalysators eine Verminderung der CO-Entwicklung durch Edelmetalldotierung erreicht werden, wie es beispielsweise in WO 98/05616 beschrieben ist. Zudem kann die Standzeit des zeolithischen Katalysators durch Hydrothermalbehandlung und gegebenenfalls nachfolgende Säurewäsche verbessert werden, wie es in WO 98/07513 und WO 95/27560 bzw. US 5,672,777 beschrieben ist.

Die Standzeit eines Fe-dotierten MFI-Zeolithen kann auch durch Wäsche mit einer Dithionit-Lösung verbessert werden, wie es in WO 98/07516 beschrieben ist.

Durch Beimischen von Inertgasen zum Reaktorfeed kann die Exothermie der Reaktion vermindert werden und die Brennbarkeit des Reaktorfeeds oder des Reaktoraustrags vermindert werden. Ein derartiges Verfahren ist in WO 98/15514 beschrieben.

Weitere geeignete Katalysatoren sind in JP-A-08 217712, JP-A-09 194412 und JP-A-100 25260 beschrieben.

Die Erfindung wird durch die nachfolgenden Beispiele näher erläutert.

### Beispiel 1

24 Nl/h N₂, 1,5 Nl/h N₂O und 0,3 ml/min Benzol wurden in einen Rohrreaktor, der 10 g eines eisenhaltigen ZBM20, hergestellt gemäß DE-A- 28 31 611, enthielt, hineingefahren. Der Austrag wurde kondensiert und anschließend ohne weitere Aufarbeitung mit einer Rate von 1,5 ml/min zusammen mit 24 Nl/h N₂ und 5 Nl/h Ammoniak in einen Rohrreaktor, der ebenfalls 10 g des ZBM20 enthielt, hineingefahren. Dabei wurde Anilin in einer Ausbeute von 1 % bezüglich Benzol erhalten.

### Beispiel 2

1,5 Nl/h N₂O und 1,5 ml/min Benzol wurden in einen Reaktor, der 10 g eines ZBM20 gemäß Beispiel 1enthielt, hineingefahren. Der gasförmige Austrag wurde anschließend ohne weitere Aufarbeitung über eine beheizte Leitung zusammen mit 10 Nl/h Ammoniak in einen Rohrreaktor, der ebenfalls 10 g des Katalysators enthielt, hineingefahren. Dabei wurde Anilin in einer Ausbeute von 10 % bezüglich N₂O erhalten.

### Beispiel 3

24 Nl/h N₂, 1,5 Nl/h N₂O, 1,5 Nl/h NH₃ und 0,3 ml/min Benzol wurden in einen Rohrreaktor, der 10 g eines ZBM20 gemäß Beispiel 1 enthielt, hineingefahren. Der Austrag wurde kondensiert. Dabei wurde Anilin in einer Ausbeute von 0,1 % bezüglich Benzol erhalten.

### Beispiel 4

24 Nl/h N₂, 1,5 Nl/h N₂O, 0,5 Nl/h NH₃ und 0,3 ml/min Benzol wurden in einen Rohrreaktor, der 10 g eines ZBM20 gemäß Beispiel 1 enthielt, hineingefahren. Der Austrag wurde kondensiert. Dabei wurde Anilin in einer Ausbeute von 0,05 % bezüglich Benzol erhalten.

### Beispiel 5

24 Nl/h N₂, 1,5 Nl/h N₂O, 2,5 Nl/h NH₃ und 0,3 ml/min Benzol wurden in einen Rohrreaktor, der 10 g eines ZBM20 gemäß Beispiel 1 enthielt, hineingefahren. Der Austrag wurde kondensiert. Dabei wurde Anilin in einer Ausbeute von 0,2 % bezüglich Benzol erhalten.

In den Beispielen 2 bis 5 wurde bei p = 1 bar (abs) und T = 350°C, in Reaktor 2 in Beispiel 2 jedoch bei 450°C gearbeitet.

## Patentansprüche

1. Verfahren zur Herstellung von aromatischen Aminen durch
(3) Gasphasen-Hydroxylierung von aromatischen C₆₋₁₄-Kohlenwasserstoffen, die zusätzlich durch 1 bis 3 C₁₋₆-Alkylreste und/oder Halogenatome substituiert sein können, mit N₂O an einem Katalysator, und ohne weitere Reinigung des hydroxylierten Produktes nachfolgende
(4) Gasphasen-Aminierung des hydroxylierten Produktes aus Stufe (1) mit Ammoniak.

2. Verfahren nach Anspruch 1, dadurch gekennzeichnet, daß in Stufe (1) ein zeolithischer Katalysator und in Stufe (2) ein silicium- und aluminiumhaltiger Katalysator eingesetzt werden.

3. Verfahren nach Anspruch 1 oder 2, dadurch gekennzeichnet, daß in Stufen (1) und/oder (2) mit Fe, Ga oder Gemischen davon dotierte Zeolithe als Katalysatoren eingesetzt werden.

4. Verfahren nach Anspruch 3, dadurch gekennzeichnet, daß als Katalysator in Stufen (1) und/oder (2) Fe-dotierte Zeolithe vom Strukturtyp MFI, MEL, MOR, BEA, MWW, FER, MTW oder MFI/MEL-Mischstrukturen eingesetzt werden.

5. Verfahren nach einem der Ansprüche 1 bis 4, dadurch gekennzeichnet, daß in Stufe (1) und Stufe (2) der gleiche Katalysator eingesetzt wird.

6. Verfahren nach einem der Ansprüche 1 bis 5, dadurch gekennzeichnet, daß das in Stufe (1) erhaltene gasförmige hydroxylierte Produkt direkt gasförmig in die Stufe (2) geführt wird.

7. Verfahren nach einem der Ansprüche 1 bis 6, dadurch gekennzeichnet, daß der aromatische Kohlenwasserstoff erst nach der in Stufe (1) erfolgten Hydroxylierung mit Ammoniak versetzt und in Stufe (2) aminiert wird.

8. Verfahren nach einem der Ansprüche 1 bis 5, dadurch gekennzeichnet, daß die Stufen (1) und (2) im selben Reaktor durchgeführt werden.

9. Verfahren nach Anspruch 8, dadurch gekennzeichnet, daß die Stufen (1) und (2) gleichzeitig durchgeführt werden.

10. Verfahren nach einem der Ansprüche 1 bis 9, dadurch gekennzeichnet, daß als aromatischer Kohlenwasserstoff Benzol, das durch 1 bis 3 C₁₋₆-Alkylreste substituiert sein kann, eingesetzt wird.
